Europäisches Patentamt

European Patent Office

Office européen des brevets

(11) Numéro de publication : **0 478 406 B1**

## (12) FASCICULE DE BREVET EUROPEEN

(45) Date de publication du fascicule du brevet :
**29.09.93 Bulletin 93/39**

(51) Int. Cl.$^5$ : **G06F 15/68**

(21) Numéro de dépôt : **91402315.5**

(22) Date de dépôt : **26.08.91**

(54) **Procédé de correction des mesures de densité optique effectuées sur un film radiographique.**

(30) Priorité : **31.08.90 FR 9010859**

(43) Date de publication de la demande :
**01.04.92 Bulletin 92/14**

(45) Mention de la délivrance du brevet :
**29.09.93 Bulletin 93/39**

(84) Etats contractants désignés :
**BE DE ES FR GB IT NL SE**

(56) Documents cités :
**FR-A- 2 555 003
US-A- 4 794 531
US-A- 4 811 090
RADIOLOGY, vol. 145. no. 3, décembre 1982,
pages 815-821; G.T. Barnes et al.:
"Radiographic Mottle and Patient Exposure in
Mammography"**

(73) Titulaire : **GENERAL ELECTRIC CGR S.A.
100, rue Camille-Desmoulins
F-92130 Issy les Moulineaux (FR)**

(72) Inventeur : **Grimaud, Michel, Cabinet
Ballot-Schmit
7, rue Le Sueur
F-75116 Paris (FR)**

(74) Mandataire : **Schmit, Christian Norbert Marie
et al
Cabinet Ballot-Schmit 7, rue Le Sueur
F-75116 Paris (FR)**

## Description

La présente invention a pour objet un procédé de correction des mesures de densité optique effectuées sur un film radiographique. Elle trouve plus particulièrement son application dans le domaine médical, et dans celui-ci notamment en mammographie. Le but de l'invention est de permettre une discrimination plus fiable de petits objets caractérisables par leur contraste, en éliminant par ailleurs les effets perturbateurs dus à la non linéarité de la courbe caractéristique de la sensibilité du film.

On connaît dans le domaine médical les examens de mammographie par lesquels on cherche à montrer des microcalcifications qui se produisent à l'intérieur du sein des patientes et qui peuvent être révélatrices de la présence de tumeurs cancéreuses. Ces microcalcifications sont des formations calciques de petite taille (25 micromètres à quelques millimètres). La densité importante des microcalcifications par rapport aux tissus environnants peut permettre leur mise en évidence sur les clichés malgré leur petite taille. On sait que par ailleurs les affections du cancer du sein peuvent être soignées à condition que le traitement soit effectué le plus tôt possible dès l'apparition des premiers signes. En conséquence, il importe de détecter la présence de ces microcalcifications bien qu'elles soient peu visibles.

On connaît, en particulier par un article "ORIGINAL INVESTIGATIONS : Computer aided detection of microcalcifications in mammogramms, Methodology and preliminary clinical study" dû à MM. Heang-Ping CHAN et al et publié dans la revue INVESTIGATIVE RADIOLOGY, en septembre 1988, volume 23, pages 664 à 671, un procédé d'assistance par ordinateur à la détection de microcalcifications. Selon ce procédé, on effectue, d'une manière connue, une numérisation d'une image radiologique révélée par un film radiographique. Puis, sur le signal numérique issu de cette numérisation, on effectue des traitements, en particulier de comparaison à un seuil, pour déterminer, statistiquement, la présence d'amas de microcalcifications dans ce cliché radiographique.

Un des problèmes essentiels à résoudre vient alors de ce que la sensibilité du film radiographique n'est pas linéaire en fonction de l'exposition qu'il a reçu. Le film reçoit une exposition. Celle-ci est l'intégrale de l'intensité du rayonnement X reçu par ce film sur le temps pendant lequel ce film a été soumis au rayonnement. Le film porte un signal de densité optique. La densité optique est le noircissement plus ou moins prononcé présent sur le cliché après développement photographique du film. Grosso modo, on peut admettre que la sensibilité du film possède trois régions. Dans une première région, la sensibilité est faible : le coefficient de correspondance des variations d'écart d'exposition reçue et des écarts associés de densité optique est faible. Cette première région correspond aux faibles valeurs d'exposition. Dans une deuxième région, où les valeurs d'exposition sont plus fortes, le coefficient de correspondance est plus fort. Dans une troisième région où l'exposition est encore plus forte, le coefficient de correspondance est à nouveau faible.

La difficulté présentée par cette situation dans le domaine de la mammographie provient de ce que le sein est composé de différents tissus ayant des coefficients d'atténuation différents. Les variations de densité optique sont liées à la présence et à la proportion de ces différents tissus. Dans les parties où l'atténuation est plus forte, le rayonnement X sera plus absorbé que dans les parties où elle est plus faible. En conséquence, l'exposition reçue par le film, à l'aplomb des parties où l'atténuation est faible, sera plus forte que l'exposition reçue à l'aplomb des parties où l'atténuation est forte.

On a pu démontrer que les microcalcifications de tailles identiques provoquent une variation de l'exposition du film qui est la même, quel que soit l'endroit où ces microcalcifications se situent dans le sein. Autrement dit, mesuré en termes d'exposition, ou plus exactement en termes du logarithme de l'exposition, ces microcalcifications présentent des contrastes identiques. Aussi, du fait de ce défaut de linéarité de la courbe de sensibilité, les microcalcifications situées dans les parties à forte ou faible atténuation schématiquement, seront révélées par des variations du signal de densité optique sur le film qui seront relativement moins grandes que les microcalcifications situées dans les parties du sein avec atténuation moyenne (et qui correspondent aux parties exposées d'une manière intermédiaire : c'est-à-dire avec la plus grande sensibilité du film).

Pour résoudre ce problème, on a découvert dans l'invention, qu'il faut établir la courbe de sensibilité du film de manière à attribuer à chaque valeur de densité optique mesurée sur le cliché une valeur équivalente correspondant au logarithme de l'exposition. Ceci est effectué par une fonction correctrice. En pratique cette fonction correctrice est l'inverse de la fonction caractérisée par la courbe caractéristique de sensibilité du film. De cette façon on gomme les effets de cette non linéarité. Il est donc nécessaire de relever la courbe caractéristique de sensibilité du film.

Il est par ailleurs connu de disposer sur le film, après l'examen mammographique, des fantômes de densités radiologiques connues. Une impression supplémentaire du film est faite à l'aide d'un appareil appelé sensitographe. L'exposition des parties du film à l'endroit de ces fantômes est alors connue. Les valeurs de densité optique du film, une fois qu'il a été ensuite développé peuvent être évaluées. A partir de ces évaluations de densité optique et de ces connaissances de l'exposition à l'endroit des fantômes, on peut par comparaison analyser le cliché. Cette technique présente cependant l'inconvénient d'être lourde à mettre en oeuvre et en pratique elle ne l'est pas. Elle est essentiellement constituée par une correction visuelle humaine instinctive, car le praticien travaille alors sur un cliché et non sur une image numérique. Quand cette technique n'est pas mise en oeuvre, la correction est induite par l'expérience de précédentes études effectuées par le praticien sur des films de même grain, et de même sensibilité théorique, ayant été soumis à des expositions similaires et supposés développés de la même façon. Cette dernière technique n'est en outre applicable que si on connaît les caractéristiques de la prise de cliché et les caractéristiques du cliché lui-même. Ceci ne peut donc pas être effectué sur un cliché, d'origine inconnue, ou non muni des traces des fantômes, qu'on chercherait néanmoins à utiliser notamment du fait de son antériorité, pour évaluer la présence préalable de ces microcalcifications.

Dans l'article cité, les auteurs n'arrivant pas à isoler les microcalcifications par une comparaison à une valeur de contraste unique sur l'ensemble du cliché, ils font appel à un procédé de seuillage adaptif. Cette technique est classique en analyse d'image. Ce procédé revient à relier la valeur de seuillage en un point à de l'information contenue dans le voisinage géographique de ce point. Dans ce cas précis, ils conditionnent le seuillage par la valeur de l'écart-type sur l'image originale, ou sur l'image filtrée spatialement (la description technique est ambiguë) dans une fenêtre de taille 51 pixels par 51 pixels, soit une fenêtre de 5.1 mm par 51 mm car leurs images sont numérisées à 100 micromètres par 100 micromètres par pixel. Ces auteurs présentent une solution permettant un meilleur seuillage mais ne cherchent pas à corriger l'effet de la variation de la sensibilité. La différence avec l'invention est importante de ce point vue. L'inconvénient de la méthode de cet article est que le fond de l'image intervient trop dans la correction.

La présente invention, dans un perfectionnement, a pour objet de remédier à ces inconvénients en proposant une technique différente par laquelle on obtient vraiment la pente de la courbe caractéristique d'un film radiographique, même alors sans avoir aucune information particulière préalable sur le type de film, ses conditions d'exposition ou de développement. Aucune présence de fantôme n'est a priori nécessaire dans l'invention. On a découvert dans l'invention que le bruit présent sur le film pouvait permettre d'estimer la pente de la courbe caractéristique de sensibilité du film qui est une représentation de la fonction de transfert entre la luminance des photons X ayant impressionné le film et le noircissement du film résultant de cette impression. Le bruit, constitue un phénomène exploratoire, en tout point du film, de la sensibilité du film à l'endroit où ce dernier se produit. Il suffit donc d'en mesurer la révélation. On en déduit la courbe caractéristique.

Il est connu dans la revue Radiology, Vol 145, N°3, pages 815-821, Décembre 1982, un article "Radiographic Mottle and Patient Exposure in mamography" dû à G.T. BARNES et D.P. CHAKRABORTY dans lequel on lie sous forme analytique la courbe caractéristique au bruit mesuré dans l'image. Mais la conclusion qui en est tirée est qu'il convient d'optimiser l'exposition de l'appareil à rayons X. Ceci n'est pas possible quand on a affaire à des clichés acquis. Ceci n'enseigne pas surtout comment calculer la courbe caractéristique elle même, mais au contraire la suppose connue.

En conséquence, l'invention a pour objet un procédé automatique de correction de la mesure de valeurs de densité optique, cette mesure étant effectuée sur un film radiographique, dans lequel

- on révèle sur un film radiographique une image d'un objet radiographié et on mesure pour chaque point du film une valeur de densité optique, cette mesure conduisant à un signal de densité optique de l'image radiographiée, comportant les étapes suivantes:
- on estime pour chaque niveau de densité optique la pente de la courbe caractéristique de la fonction de transfert réalisée par le film, cette courbe caractéristique établissant la correspondance entre l'exposition radiographique que le film a reçue et la densité optique qui en résulte,
- on transforme le signal de densité optique de l'image radiographiée par une opération de filtrage spatial suivie par un filtrage en chapeau haut de forme pour obtenir une image transformée en densité optique
- on dresse, point par point du film, la carte des pentes de cette courbe caractéristique, en attribuant à chaque point de l'image une valeur de pente de cette courbe, cette valeur de pente étant fonction de cette courbe et de la valeur de densité optique en ce point,
- et on corrige dans l'image transformée la mesure de densité optique en chaque point de cette image avec cette carte des pentes de façon qu'à chaque point on attribue une valeur égale au rapport de cette valeur de densité optique et de la pente de la courbe caractéristique en ce point.

L'invention sera mieux comprise à la lecture de la description qui suit et l'examen des figures qui l'accompagnent. Celles-ci ne sont donné qu'à titre indicatif et nullement limitatif de l'invention. Les figures montrent :

- figure 1 : une représentation schématique d'un dispositif utilisable pour mettre en oeuvre le procédé de l'invention ;
- figure 2 : l'allure du signal de densité optique mesuré ;
- figures 3 et 4 : respectivement la pente de la courbe caractéristique du film et son assimilation à la mesure du bruit permettant une mise en oeuvre de l'invention ;

- figures 5a à 5i : des diagrammes de représentation des effets physiques mis en oeuvre dans l'invention ;
- figure 6 : un organigramme du traitement selon l'invention ;
- figures 7a à 7d : des traitements effectués sur des mesures de bruit pour permettre le calcul de la courbe caractéristique ;
- figure 8 : un diagramme schématique permettant la compréhension des traitements de signaux effectués dans l'invention.

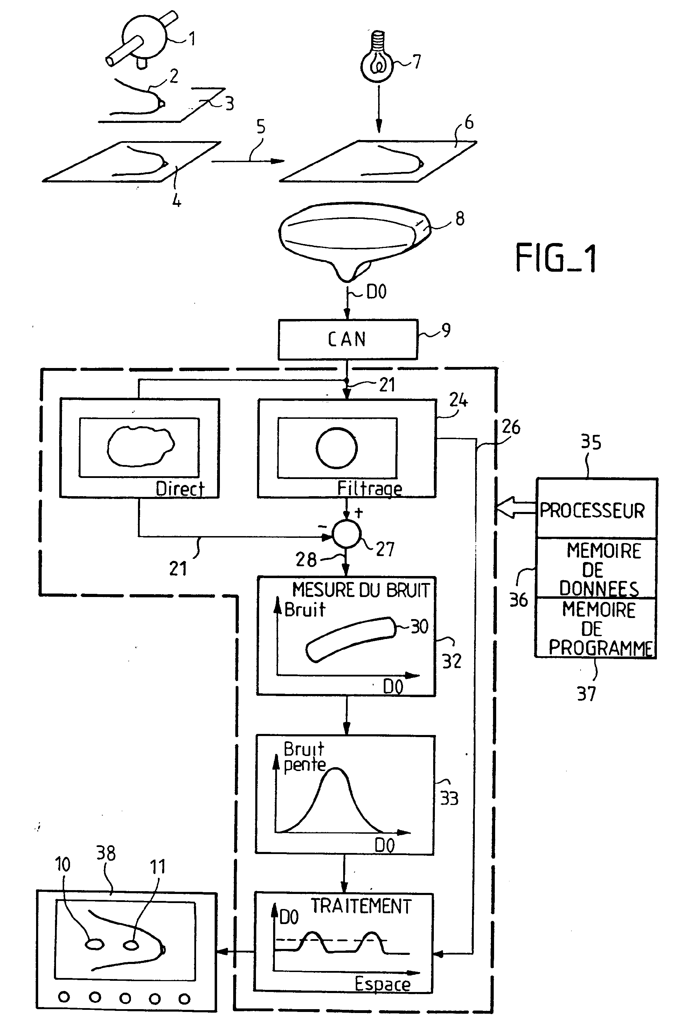

La figure 1 montre un dispositif utilisable pour la mise en oeuvre du procédé de l'invention. Elle montre une radiographie, au moyen d'un tube à rayons X 1, d'un sein 2 placé sur un plateau porte-sein 3 en vue d'impressionner un film photosensible 4 situé en-dessous du plateau 3. Après un traitement chimique de type classique 5, le film radiographique 4 est développé en un cliché 6. Ce cliché est observé d'une manière connue pour être numérisé.

Le principe de la numérisation consiste à illuminer le cliché 6 avec une source lumineuse 7 et à observer, au moyen d'un photorécepteur 8, l'image de densité optique montrée par le cliché 6. Le signal DO délivré par le photorécepteur 8, qui est un signal évoluant dans le temps en fonction de la scrutation des lignes de l'image, est numérisé dans un convertisseur analogique numérique 9. La mesure, pour chaque point du cliché 6 d'une valeur de densité optique peut donc être considérée comme effectuée par le photorécepteur 8. Le signal de densité optique de l'image radiographiée, en particulier quand il est numérisé, peut être considéré comme celui qui sort du convertisseur analogique numérique 9.

Dans la suite de cette description, on montrera les effets physiques des traitements à effectuer sur le signal numérique (noté DO, lui aussi) délivré par le convertisseur 9. Il est cependant entendu que ces traitements sont effectués dans un ordinateur 35-37 qui traite des informations numériques, codées normalement sans forme binaire, correspondant à ces images en fonction d'un programme que l'on va expliquer ci-après. L'image produite après traitement peut être montrée sur un moniteur 38.

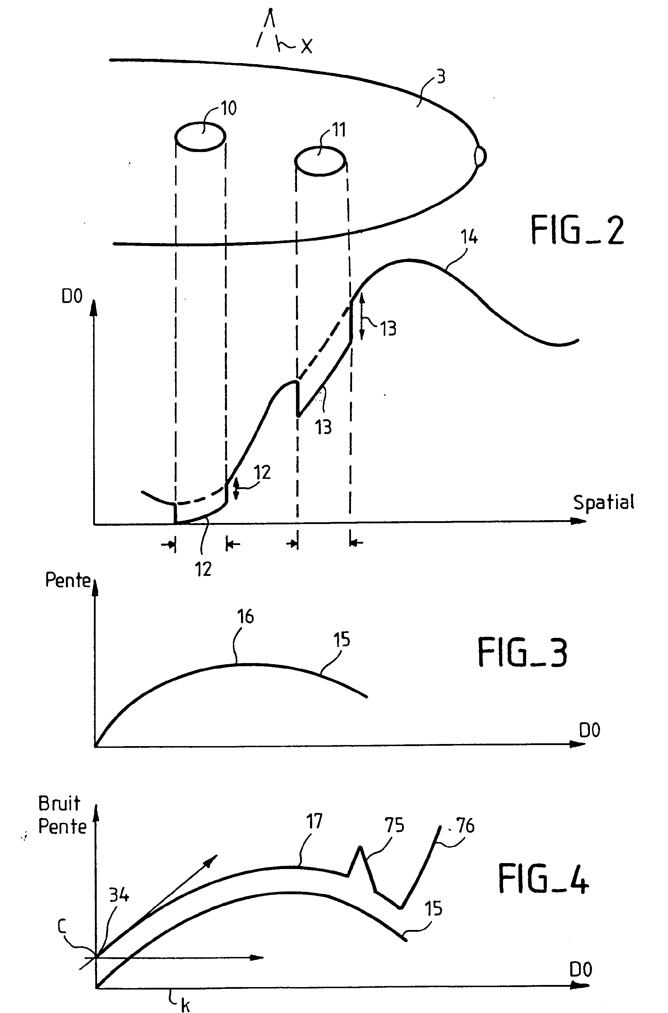

Sur la figure 2, on a schématiquement représenté, en dessous du sein irradié 3, les conséquences de la variation de la sensibilité du film en fonction de la nature de l'exposition. La courbe de la figure 2 montre pour une ligne d'image du cliché 6 le signal 14 de densité optique mesuré DO, en fonction de l'abscisse "spatiale" d'un point mesuré sur cette ligne. Ce signal 14 présente un profil fluctuant en fonction du coefficient d'atténuation des tissus interposés. Deux microcalcifications 10 et 11, supposées exactement identiques, provoquent des variations respectivement 12 et 13 différentes du signal de densité optique sur le cliché 6. Ces variations différentes du signal de densité optique sont dues à des sensibilités différentes du film à l'endroit où elles sont révélées. Ces variations de sensibilité sont causées en majeure partie par la composition différente des tissus se trouvant au-dessus et au-dessous des microcalcifications 10 et 11.

La figure 3 montre la courbe 15 de la pente de la courbe caractéristique de sensibilité du film. Cette pente 15 présente un sommet 16 correspondant à l'endroit de la courbe de sensibilité où celle-ci est la plus raide. Dans l'invention, on a découvert que si on mesurait le bruit on pouvait choisir un estimateur de bruit propre au cliché 6 dont la courbe caractéristique 17 (figure 4) est tout à fait comparable à la pente de la courbe caractéristique 15 du cliché 6. Ces deux courbes se déduisent l'une de l'autre par une constante additive C dont on montrera plus loin comment on peut calculer la valeur. Donc, dans l'invention, on évalue la pente de la courbe caractéristique de la fonction de transfert réalisé par le film en élaborant, pour chaque niveau de densité optique DO, un estimateur de bruit de ce signal de densité optique.

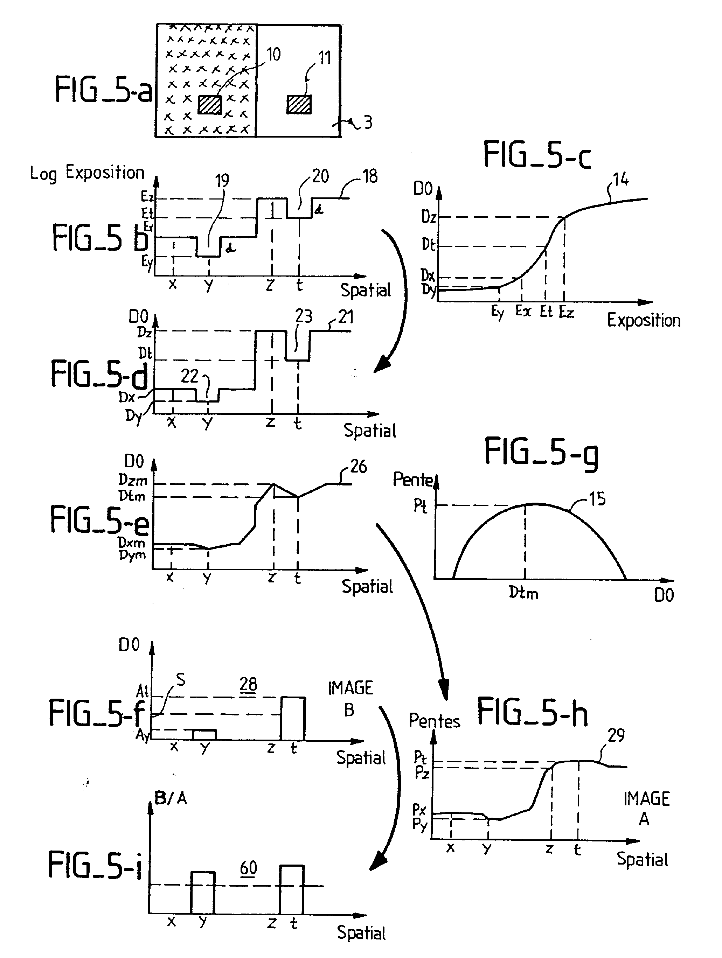

Les figures 5a à 5i montrent d'une manière schématisée les étapes du procédé de l'invention. Dans celles-ci, le sein 3 et les microcalcifications 10 et 11 sont représentés par les surfaces rectangulaires. Ces surfaces rectangulaires conduisent schématiquement à des signaux de densité optiques impulsionnels. Sur la figure 5b on a représenté, pour la densité des tissus du sein de la figure 5a, les différentes expositions auxquelles est soumis le film 4 à des abscisses spatiales différentes x, y, z, t. On constate que les microcalcifications 10 et 11 retentissent, dans le signal d'exposition 18 montré, en deux créneaux négatifs 19 et 20 de même hauteur. Par contre, figure 5c, du fait de l'existence de la courbe caractéristique 14 du film 4, les densités optiques observables sur le cliché 6 mènent à un signal de densité optique 21, figure 5d, possédant deux créneaux négatifs respectivement 22 et 23 dont la caractéristique essentielle est qu'ils sont eux de hauteurs différentes.

Ils correspondent donc à des accidents de même nature et retentissent dans le signal sous des formes différentes. L'image dite originale en densité optique de la figure 5d peut être transformée par une opération de filtrage médian spatial 241 suivi d'une opération de moyennage spatial 242. L'opération de filtrage médian spatial 241 consiste pour l'essentiel à attribuer, pour chaque abscisse de la courbe 21, un autre signal de densité optique égal à la valeur intermédiaire d'une liste, ordonnée en valeur croissante, de valeurs de densité optique DO mesurées pour des points voisins du point étudié. Plutôt que la valeur intermédiaire on pourrait aussi prendre la valeur correspondant au premier ou au dernier quartile ou encore à toute autre subdivision. Ce filtrage médian transforme le signal de la courbe 21 en un autre signal 25. Le moyennage spatial 242 consiste à attribuer, pour chaque abscisse de cette autre courbe, un nouveau signal 26 de densité optique égal à la moyenne de ces autres signaux des densités optiques obtenus précédemment à des localisations spatiales voisines de celle de cette abscisse. Le voisinage de la moyenne spatiale du filtre 242 est typiquement un voisinage de 20 X 20 pixels. Cette opération globale qui est effectuée dans un filtre 24 (figure 1) conduit à une forme plus lisse 26 de l'image des contours du sein 3. Le signal filtré 26 est représenté sur la figure 5e.

En faisant la soustraction, dans un soustracteur 67, du signal 25 filtré par le filtre médian 241 et de ce signal 25 filtré par un filtre 68 effectuant une fermeture, on peut obtenir un signal 28 en densité optique du contraste des microcalcifications. Ce filtrage 68 de fermeture sera expliqué plus loin. Le signal 28 est représenté sur la figure 5f. Sans le traitement de l'invention, on voit que la comparaison du signal 28 à un seuil de valeur S ne permet de mettre en évidence automatiquement que la microcalcification 11 : la microcalcification 10 insuffisamment révélée passera inaperçue. Avec l'invention, on modifie le signal 28 de telle manière que le seuil S puisse être comparé d'une manière homogène aux créneaux correspondant aux microcalcifications 10 et 11.

On relève, conformément à ce qui a été dit précédemment, une pente de la courbe caractéristique fonction de la sensibilité du film exprimé en densité optique, figure 5g. Elle est extraite du bruit d'image. On dresse alors, figure 5h, la carte 29 des pentes en chaque point de l'image. Pour élaborer la carte 29, on procède de préférence de la manière suivante. Pour une abscisse donnée d'un point, par exemple l'abscisse t, on prélève sur une courbe représentant le phénomène, par exemple la courbe 26, une valeur de densité optique Dtm associée. Sur la courbe des pentes de la figure 5g on relève alors une pente Pt associée à l'abscisse Dtm. On construit alors la carte 29 des pentes en associant à l'abscisse spatiale t une ordonnée Pt déduite de la courbe 15 (figure 5g) comme correspondant à l'abscisse Dtm. On pourrait aussi établir la carte 29 des pentes à partir de l'image originale ayant seulement subi le filtrage médian ou même à partir de l'image originale non filtrée (courbe 21, figure 5d).

On appelle image A la carte des pentes 29. On appelle image B la courbe 28 obtenue point à point à la sortie du soustracteur 67. On élabore alors l'image 60 corrigée B/A dans une opération 69, figure 5i, en combinant l'image B et l'image A de façon qu'à chaque abscisse commune à ces deux images on attribue une ordonnée égale au rapport des ordonnées attribuées dans chacune de ces deux images.

Le signal 60 permet la détection automatique des microcalcifications par comparaison au seuil S, ou par comparaison à un seuil tenant compte de la correction elle même si celle-ci n'est pas normalisée.

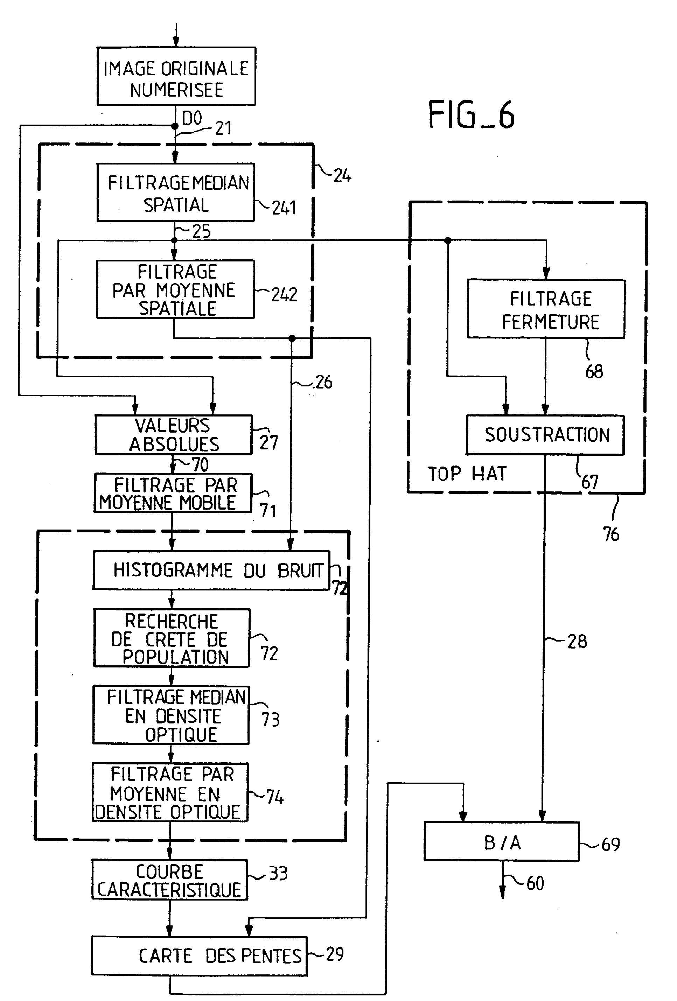

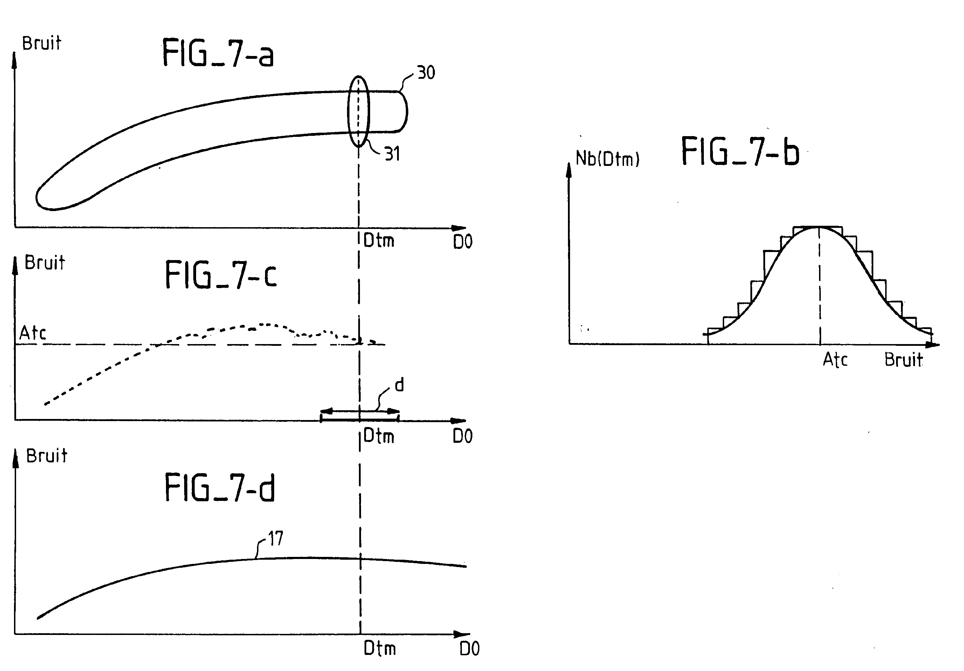

Le procédé qui est ainsi décrit figures 5a à 5i est le procédé de l'invention. Il est perfectionné par une étape consistant à élaborer la courbe 15 des pentes à partir d'un estimateur du bruit. L'élaboration de cet estimateur du bruit va être expliquée dans son principe en référence aux figures 6 et 7a à 7e. Cet estimateur est élaboré principalement à partir d'un signal 70 disponible en sortie d'un soustracteur 27. On relève pour chacune des lignes de l'image sur le cliché 6 les signaux de soustraction 70. Ces signaux 70 sont redressés de manière à ne considérer que leur valeur absolue. C'est pourquoi le soustracteur 27 est dit un soustracteur en valeur absolue. On notera que sur le plan pratique, en traitement numérique, cette prise en compte des valeurs absolues est simple. En effet, la soustraction numérique de deux signaux conduit à un résultat numérique associé à un bit de signe. Il suffit dans le cas présent, tout simplement, de ne pas tenir compte du bit de signe. La population statistique étudiée comporte donc un ensemble de couples de valeurs. Les valeurs de ces couples concernent d'une part les valeurs absolues des différences de densités optiques et, d'autre part, les adresses spatiales où ces valeurs absolues ont été relevées. Plutôt que d'utiliser directement le signal 70 on préfère, pour homogénéiser les bruits à petite échelle, effectuer un filtrage 71, par moyenne mobile. Pour des pixels de 50 micromètres X 50 micromètres, on attribue ainsi, au pixel central d'une fenêtre de 10 pixels par 10 pixels, la moyenne des valeurs absolues des pixels de la fenêtre. Le signal de valeurs absolues, filtré ou non, est représentatif du bruit seul puisque le filtrage médian 241 avait pour objet d'éliminer ce bruit du signal d'image. Par soustraction avec ce signal d'image, le bruit réapparaît donc seul.

On utilise ensuite de préférence la courbe 26 (on pourrait utiliser en pratique une autre courbe) pour retenir une correspondance entre une adresse spatiale et une valeur de densité optique filtrée. La figure 6 montre ainsi que les valeurs absolues sont obtenues par soustraction des valeurs originales des valeurs originales filtrées par le filtre médian uniquement. Ainsi à l'adresse t vue précédemment on a associé une valeur absolue At. Sur la courbe 26 à la même adresse t on avait associé une densité optique, filtrée par filtre médian et moyenne, de valeur Dtm. On dresse alors en une opération 72 un histogramme du bruit. La population statistique concernée est la population constituée des couples Dtm-At relatifs à une même adresse spatiale t. Cette population statistique est portée sur un diagramme, figure 7a : les valeurs absolues At sont portées en ordonnée comme étant représentatives du bruit. Les densités optiques filtrées par le filtre médian et la moyenne sont portées en abscisse. On effectue ce travail pour tous les points d'image du cliché 6. En pratique on pourrait se limiter à une proportion inférieure de ces nombres de points d'image. Ceci aurait pour effet de rendre cependant les calculs statistiques ultérieurs moins fiables.

L'essence du perfectionnement de l'invention se situe dans le fait que les événements de cette population statistique se répartissent alors, sur le diagramme de la figure 7a, en un nuage 30 ayant sensiblement la forme de la pente de la courbe caractéristique 15 de sensibilité du film. C'est d'ailleurs ce qui a conduit à la découverte de l'invention. On observe alors, en reprenant l'exemple précédent, pour une densité optique moyenne Dtm un ensemble 31 de valeurs possibles de At. En effet, on a obtenu plusieurs fois la valeur Dtm, et à chaque fois, on a obtenu des valeurs At qui n'ont aucun raison d'être identiques.

On s'est intéressé à la répartition statistique de ces différentes valeurs At de bruit réparties en un certain nombre de gammes. On s'est rendu compte alors que le nombre N des occurrences dans chaque gamme des valeurs At avait une forme en cloche, représentée sur la figure 7b, de part et d'autre d'une valeur crête Atc. La figure 7b montre, pour la densité optique Dtm, en abscisse les différentes valeurs possibles de At, et en ordonnée le nombre Nb d'événements de la population situés à cette valeur. On remplace alors en une étape 72 le nuage 30 de la figure 7a par une courbe représentée par la figure 7c dans laquelle, à chaque abscisse Dtm, on a affecté une ordonnée Atc correspondant à la crête de population de la figure 7b.

On constate que la courbe de la figure 7c n'est pas une courbe lisse. Pour utiliser une courbe acceptable, on transforme alors la suite non continue des points de la courbe de la figure 7c par un filtrage médian 73 suivi d'un moyennage 74, tous les deux pris sur un voisinage mesuré le long de l'axe des densités optiques. Dans un exemple simple avec le filtre médian 73, à chaque abscisse Dtm on remplace la valeur Atc trouvée par une autre valeur Atm égale à la valeur médiane d'un ensemble de valeurs trouvées sur un segment $\underline{d}$ autour de Dtm. On pourrait là aussi choisir un autre type de filtrage. Il est important cependant que ce filtrage soit effectué en fonction d'un voisinage mesuré le long de l'axe des densités optiques. Puis on remplace ces autres valeurs Atm, filtrées d'une manière médiane, par des nouvelles valeurs Atn obtenues par moyennage des valeurs Atm trouvées à des abscisses voisines de l'abscisse Dtm, et situées elles aussi sur un segment de longueur $\underline{d}$ centrée sur Dtm. On n'est cependant pas obligé de prendre deux fois le même $\underline{d}$. Dans l'invention, $\underline{d}$ vaut 5, la valeur maximum de Dtm valant 100. Cette opération est résumée en 32 sur la figure 6. Le résultat de ce traitement est visible sous la forme de la courbe 17 sur les figures 7d et 4.

L'opération 32 de mesure de la courbe de bruit 17, est suivie d'une opération 33, non nécessaire à priori, au cours de laquelle on calcule la constante C permettant de mesurer le décalage entre la courbe 17 mesurée et la courbe vraie 15. Cette opération n'est pas nécessaire puisqu'on peut, dans une application dégradée de la méthode négliger le calcul de C. La pente de la véritable courbe caractéristique 15 est théoriquement nulle à l'origine des densités optiques. Malheureusement, pour un cliché ordinaire, on ne connaît pas la valeur du bruit à l'origine. On va alors estimer cette valeur en considérant que, pour les faibles valeurs de densité optique, la courbe représentative de l'estimateur de bruit est assimilable à une droite. Par une méthode numérique de régression linéaire on calcule alors l'ordonnée du point d'instruction de la courbe 17 avec l'axe des ordonnées. Dans ce but, on effectue un histogramme cumulé de l'image originale. On cherche alors les deux niveaux de gris dont les valeurs dans l'histogramme cumulé sont respectivement 1% et 5% du nombre total de pixels dans l'image. On ne conserve alors de la fonction de bruit que l'intervalle compris entre ces deux valeurs. On cherche ensuite, par la méthode des moindres carrés, la droite approximant cette fonction de bruit réduite. Soit $y_{est} = ax+b$ l'équation de cette droite. On a un ensemble de couplet $(x_i, y_i)$ ou $x_i$ représente un niveau de gris appartenant à l'intervalle précédemment défini et $y_i$ la valeur de bruit correspondant à ce niveau de gris. La méthode des moindres carrés vise à minimiser la quantité

$$\Sigma_i(y_{est}(x_i) - y_i)^2 = \Sigma_i(ax_i + b - y_i)^2 \quad (1)$$

Minimiser cette quantité revient à annuler les dérivées premières de (1) par rapport à a et b, soit encore à résoudre le système d'équation suivant :

$$\Sigma_i(ax_i + b - y_i) = 0$$
$$\Sigma_i x_i(ax_i + b - y_i) = 0$$

Ce système de deux équations à deux inconnus permet de trouver la valeur des coefficients a et b. b est alors la valeur de la constante soustractive C reliant l'estimateur de bruit et la pente de la courbe caractéristique.

La courbe 17 comporte des artefacts 75 et 76 aux grandes valeurs d'exposition. Ces artefacts sont dus à la numérisation du film dans les zones très opaques. Ceci n'est pas gênant puisqu'elles sont situées en dehors de la zone d'intérêt pratique du film.

Toutes les opérations statistiques sont mises en oeuvre par un processeur 35 de l'ordinateur 35-37. Cet ordinateur comporte une mémoire 36 dans laquelle on peut stocker les images délivrées par le convertisseur 9 ainsi que, au moins d'une manière temporaire, les images de travail élaborées au cours du traitement, le nuage 30 et la courbe 17. L'ordinateur comporte encore une mémoire de programme 37 comportant la suite des instructions permettant d'effectuer les traitements évoqués jusqu'ici. Le processeur 35 peut également gérer le convertisseur 9 et le moniteur de visualisation 38 sur lequel in fine on peut faire apparaître les microcalcifications 10 et 11 préalablement évoquées. Un bus non représenté permet au processeur 35 de diriger toutes les opérations, depuis l'illumination du cliché 6 jusqu'à la visualisation sur le moniteur 38.

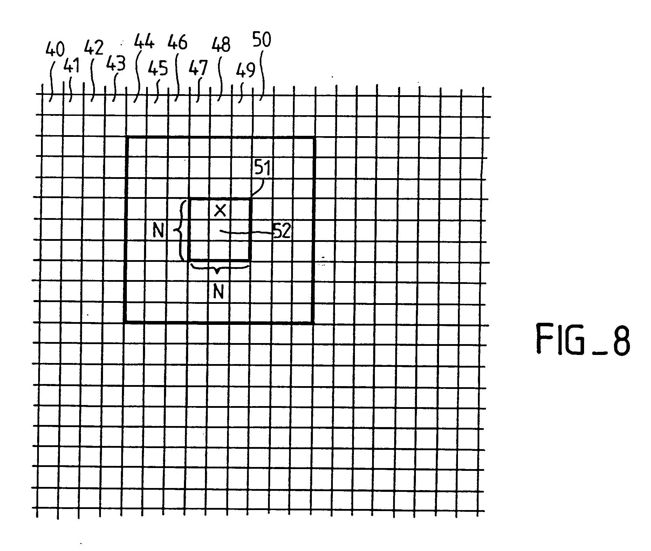

On va donner maintenant un aperçu des filtrages préférentiels mis en oeuvre dans l'invention pour élaborer le signal 28 . La figure 8 montre des points d'image tels que 40 à 50 du cliché 6. Ces points d'image sont scrutés par le photorécepteur 8 et le signal élaboré par le photorécepteur 8 est ensuite échantillonné par le convertisseur 9 qui donne à chacun de ces points d'image les valeurs de densité optique mesurée. Le filtrage effectué dans le filtre médian 241 permet d'élaborer l'image de densité optique filtrée d'une manière médiane en traitant les densités optiques de N x N (N de préférence impair) points d'image contenus dans une fenêtre mobile 51. Dans l'exemple décrit la taille de la fenêtre 51 est de 3 x 3. Le résultat de ce traitement est attribué au point d'image 52 situé au centre de cette fenêtre mobile 51.

En ce qui concerne le filtrage 68 et la soustraction 67, ceux-ci peuvent consister en un filtrage 76 connu dit en chapeau haut de forme (top hat) comportant une fermeture au sens des transformations de morphologie mathématique. Cette fermeture est suivie de la soustraction. Cette fermeture comporte une dilatation suivie d'une érosion, toutes les deux de préférence avec le même élément structurant : une fenêtre de 9 x 9 pixels dans un exemple. De préférence cependant dans l'invention on fait un filtrage en chapeau haut de forme spécial dit par reconstruction numérique. Dans ce filtrage spécial on effectue l'érosion d'une manière itérative en l'appliquant, pour une érosion suivante, sur les résultats d'une érosion précédente. On réitère cette opération jusqu'à ce qu'on puisse considérer que l'image n'évolue plus d'une itération à l'autre. L'intérêt de cette transformation avec reconstruction numérique est alors de conduire à une bien meilleure détection des objets fortement contrastés que ne le fait la transformation chapeau haut de forme normale. Ces transformations spéciales sont en particulier décrites dans le livre "Précis d'analyse d'image". Presses du CNRS, ayant pour auteurs M. COSTER et J.L CHERMANT, 1988.

La fenêtre de 9 X 9 pixels permet de sélectionner des microcalcifications de taille donnée. Comme les microcalcifications sont de tailles différentes on met par exemple trois fois en oeuvre le calcul de correction 69. Une première fois avec une fenêtre du filtrage 68 égale à 9 X 9, une deuxième fois avec une fenêtre 15 X 15 et une troisième fois avec une fenêtre 27 X 27. On peut montrer qu'avec ces trois images on balaye toute l'étendue possible des tailles des microcalcifications.

## Revendications

1. Procédé automatique de correction de la mesure (8) de valeurs de densité optique, cette mesure étant effectuée sur un film radiographique (6), dans lequel
   - on révèle sur un film (6) radiographique une image (10,11) d'un objet (3) radiographié et on mesure pour chaque point du film une valeur de densité optique (DO), cette mesure conduisant à un signal de densité optique de l'image radiographiée, comportant les étapes suivantes:
   - on estime pour chaque niveau de densité optique la pente de la courbe caractéristique de la fonction de transfert (15) réalisée par le film, cette courbe caractéristique établissant la correspondance entre l'exposition radiographique que le film a reçue et la densité optique qui en résulte,
   - on transforme le signal de densité optique de l'image radiographiée par une opération de filtrage spatial suivie par un filtrage en chapeau haut de forme pour obtenir une image transformée (B) en densité optique
   - on dresse, point par point du film, la carte (A) des pentes de cette courbe caractéristique, en attribuant à chaque point de l'image une valeur de pente de cette courbe, cette valeur de pente étant fonction de cette courbe et de la valeur de densité optique en ce point,
   - et on corrige dans l'image transformée (B) la mesure de densité optique en chaque point de cette image avec cette carte des pentes (A) de façon qu'à chaque point on attribue une valeur égale au rapport de cette valeur de densité optique et de la pente de la courbe caractéristique en ce point.

2. Procédé selon la revendication 1 caractérisé en ce que
   - on élabore, pour chaque niveau de densité optique, un estimateur (30) de bruit du signal de densité optique, et
   - on déduit (33) la pente de la courbe caractéristique de cet estimateur.

3. Procédé selon la revendication 2, caractérisé en ce que pour élaborer l'estimateur de bruit
   - on calcule, pour chaque point d'image, la valeur absolue (At) de la différence entre le signal de densité optique de l'image radiographique révélée et le résultat d'un filtrage spatial de ce signal.

4. Procédé selon la revendication 3, caractérisé en ce que
   - on filtre spatialement le signal de densité optique de l'image radiographique relevée avec un filtre médian,
   - ce filtre médian attribuant, à chaque point choisi d'image, une valeur de densité optique qui correspond à une valeur de rang N une fois qu'on a retenu, et rangé dans un ordre croissant, 2N+1 valeurs de densité optique pour 2N+1 points situés autour du point choisi (3 x 3) et le comprenant.

5. Procédé selon l'une quelconque des revendications 1 à 4, caractérisé en ce que pour élaborer un estimateur pour chaque niveau de densité optique
    - on recherche la moyenne des valeurs de densité optique des points d'image appartenant à un voisinage (20 x 20), et
    - on attribue au point situé au centre de ce voisinage comme nouvelle valeur de densité optique cette moyenne des valeurs,
    - on établit un histogramme (30) à double entrée de cette nouvelle valeur de densité optique et d'un estimateur de bruit qui correspondent au même point.

6. Procédé selon la revendication 4, caractérisé en ce que pour évaluer la courbe caractéristique,
    - on recherche, pour chaque niveau de densité optique, la valeur de bruit la plus représentée (Atc), et
    - on filtre (73-74) cette valeur la plus représentée en fonction du niveau de densité optique.

7. Procédé selon l'une quelconque des revendications 1 à 6, caractérisé en ce que pour déduire la pente de la courbe caractéristique
    - on soustrait une constante (C) à la valeur de l'estimateur.

8. Procédé selon la revendication 7, caractérisé en ce que pour soustraire la constante on la calcule par régression linéaire sur un ensemble de valeurs de l'estimateur correspondant à de faibles valeurs de densité optique.

9. Procédé selon l'une quelconque des revendications 1 à 8 caractérisé en ce que pour corriger la mesure de densité optique en chaque point
    - on effectue une transformation (76) chapeau haut de forme, avec une taille donnée (9X9) d'élément structurant, sur le signal (25) de densité optique de l'image radiographié filtré (241).

10. Procédé selon la revendication 9 caractérisé en ce qu'on réitère la correction pour une autre taille (15X15) donnée de l'élément structurant.

11. Procédé selon la revendication 9 ou la revendication 10 caractérisé en ce qu'on effectue la transformation chapeau haut de forme par reconstruction numérique.

**Patentansprüche**

1. Automatisches Verfahren zur Korrektur der Messung (8) von optischen Dichtewerten, wobei die Messung an einem Röntgenfilm (6) ausgeführt wird, bei dem
    - von einem Röntgenfilm (6) ein Bild (10, 11) eines geröntgten Objektes (3) entwickelt wird und für jeden Punkt des Films ein optischer Dichtewert (DO) gemessen wird, wobei die Messung zu einem optischen Dichtesignal des geröntgten Bildes führt,
    umfassend die folgenden Schritte:
    - für jeden optischen Dichtepegel wird die Steigung der Kennlinie der durch den Film ausgeführten Übertragungsfunktion (15) geschätzt, wobei diese Kennlinie die Entsprechung zwischen der Röntgenbelichtung herstellt, die der Film empfangen hat, und der optischen Dichte, die sich daraus ergibt,
    - das optische Dichtesignal des geröntgten Bildes wird durch eine räumliche Filteroperation umgewandelt, der eine Zylinderhutform-Filterung folgt, um ein in optische Dichte umgewandeltes Bild (B) zu erhalten,
    - Filmpunkt für Filmpunkt wird die Karte (A) der Steigungen der Kennlinie erstellt, wobei jedem Punkt des Bildes ein Steigungswert dieser Kurve zugeordnet wird, wobei der Steigungswert von der Kurve und dem optischen Dichtewert in diesem Punkt abhängt,
    - und in dem transformierten Bild (B) wird die optische Dichtemessung in jedem Punkt des Bildes mit der Karte der Steigungen (A) korrigiert derart, daß jedem Punkt ein Wert gleich dem Verhältnis des optischen Dichtewertes und der Steigung der Kennlinie in dem Punkt zugeordnet wird.

2. Verfahren nach Anspruch 1, dadurch **gekennzeichnet**, daß
    - für jeden optischen Dichtepegel eine Rausch-Schätzfunktion (30) des optischen Dichtesignals ausgearbeitet wird und
    - die Steigung der Kennlinie der Schätzfunktion abgeleitet wird (33).

3. Verfahren nach Anspruch 2, dadurch **gekennzeichnet**, daß zur Ausarbeitung der Rausch-Schätzfunktion
    - für jeden Bildpunkt der Absolutwert (At) der Differenz zwischen dem optischen Dichtesignal des entwickelten Röntgenbildes und dem Ergebnis einer räumlichen Filterung dieses Signals berechnet wird.

**4.** Verfahren nach Anspruch 3, dadurch **gekennzeichnet**, daß

- das optische Dichtesignal des entwickelten Röntgenbildes mit einem Medianfilter räumlich gefiltert wird,
- wobei der Medianfilter jedem ausgewählten Bildpunkt einen optischen Dichtewert zuordnet, der einem Wert vom Rang N entspricht, wenn er einmal gehalten worden ist, und in eine höhere Ordnung klassifiziert wird, wobei 2N+1 optische Dichtewerte für 2N+1 Punkte um den ausgewählten Punkt (3 x 3) liegen und diesen enthalten.

**5.** Verfahren nach einem beliebigen der Ansprüche 1 bis 4 dadurch **gekennzeichnet**, daß zur Ausarbeitung einer Schätzfunktion für jeden optischen Dichtepegel

- der Mittelwert der optischen Dichtewerte der zu einer Umgebung (20 x 20) gehörenden Bildpunkte gesucht wird und
- dem im Zentrum dieser Umgebung liegenden Punkt dieser Mittelwert als neuer optischer Dichtewert zugeordnet wird,
- ein Histogramm (30) mit doppelter Eingabe des neuen optischen Dichtewertes und einer Rausch-Schätzfunktion vorgesehen wird, die demselben Punkt entsprechen.

**6.** Verfahren nach Anspruch 4, dadurch **gekennzeichnet**, daß zur Auswertung der Kennlinie

- für jeden optischen Dichtepegel der am meisten dargestellte Rauschwert (Atc) gesucht wird und
- dieser am meisten dargestellte Wert abhängig vom neuen optischen Dichtepegel gefiltert wird (73-74).

**7.** Verfahren nach einem beliebigen der Ansprüche 1 bis 6, dadurch **gekennzeichnet**, daß zur Ableitung der Steigung der Kennlinie,
  - eine Konstante (C) vom Wert der Schätzfunktion subtrahiert wird.

**8.** Verfahren nach Anspruch 7, dadurch **gekennzeichnet**, daß zur Subtraktion der Konstanten diese durch lineare Regression an einer Menge von Werten der Schätzfunktion entsprechend kleinen optischen Dichtewerten berechnet wird.

**9.** Verfahren nach einem beliebigen der Ansprüche 1 bis 8, dadurch **gekennzeichnet**, daß zur Korrektur der optischen Dichtemessung in jedem Punkt,
  - eine Zylinderhutform-Transformation (76) mit einer gegebenen Strukturelement-Größe (9x9) an dem optischen Dichtesignal (25) des gefilterten Röntgenbildes (241) ausgeführt wird.

**10.** Verfahren nach Anspruch 9, dadurch **gekennzeichnet**, daß die Korrektur für eine weitere gegebene Größe des Strukturelementes (15x15) wiederholt wird.

**11.** Verfahren nach Anspruch 9 oder Anspruch 10, dadurch **gekennzeichnet**, daß die Zylinderhutform-Transformation durch Digitalrekonstruktion ausgeführt wird.

**Claims**

**1.** An automatic method for correcting the measurement (8) of values of optical density, the said measurement being carried out on a radiographic film (6), in which
  - an image (10, 11) of a radiographed object (3) is developed on a radiographic film (6), and a value of optical density (DO) is measured for each point on the film, this measurement leading to a signal of optical density of the radiographed image,

including the following steps:
  - the gradient of the characteristic curve of the transfer function (15) for the film is estimated for each level of optical density, this characteristic curve establishing the correspondence between the radiographic exposure which the film has received and the optical density that results therefrom,
  - the signal of optical density of the radiographed image is converted by a spatial filtering operation followed by a filtering operation of top hat form for obtaining an image (B) converted in optical density,
  - the chart (A) of the slopes of this characteristic curve is drawn up, point by point of the film, by attributing one value of gradient of the said curve to each point of the image, the said value of gradient being a function of the said curve and of the value of optical density at that point,
  - and the measurement of optical density at each point on the said image is corrected in the converted image (B) with the said gradient chart (A), whereby a value is attributed to each point which is equal to the ratio of the said value of optical density to the gradient of the characteristic curve at that point.

**2.** A method according to Claim 1, characterised in that
  - an estimation (30) of optical density signal noise is produced for each optical density level, and
  - the gradient of the characteristic curve of this estimation is deduced (33).

3. A method according to Claim 2, characterised in that, for the purpose of producing the noise estimation
   - the absolute value (At) of the difference between the optical density signal of the revealed radiographic image and the result of a spatial filtering operation on the said signal is calculated for each point on the image.

4. A method according to Claim 3, characterised in that
   - the optical density signal of the revealed radioactive image is spatially filtered with a median filter,
   - the said median filter attributes to each selected point on the image a value of optical density which corresponds to a serial number N, once 2N + 1 values of optical density, for 2N + 1 points situated around the selected point (3 x 3) and including it, have been retained and arranged in an increasing order.

5. A method according to any one of Claims 1 to 4, characterised in that, for the purpose of producing an estimation for each level of optical density
   - the mean of the values of optical density of the points on the image relating to one location (20 x 20) is searched for, and
   - the said mean value is attributed to the point situated at the centre of the said location, as a new value of optical density,
   - a double input histogram (30) of the said new value of optical density, and a noise estimation, are established corresponding to the same point.

6. A method according to Claim 4, characterised in that, in order to evaluate the characteristic curve,
   - the noise value (Atc) appearing most commonly is searched for in respect of each optical density level, and
   - this commonest value is filtered (73 - 74) as a function of the optical density level.

7. A method according to any one of Claims 1 to 6, characterised in that, in order to deduce the slope of the characteristic curve
   - a constant (C) is subtracted from the value of the estimation.

8. A method according to Claim 7, characterised in that, in order to subtract the constant, it is calculated by linear regression on a set of values of the estimation corresponding to low values of optical density.

9. A method according to any one of Claims 1 to 8, characterised in that, in order to correct the measurement of optical density at each point
   - a conversion (76) of top hat form is effected, with a given size (9 x 9) of structuring element, on the signal (25) of optical density of the filtered radiographed image (241).

10. A method according to Claim 9, characterised in that the correction is reiterated for another given size (15 x 15) of the structuring element.

11. A method according to Claim 9 or Claim 10, characterised in that the conversion of top hat form is effected by digital reconstruction.

FIG_1

FIG_2

X

3

10

11

14

D0

13

13

12

12

12

Spatial

Pente

16

15

FIG_3

D0

Bruit
Pente

17

75

76

34

C

15

FIG_4

D0

k

FIG_5-a

FIG_5-c

FIG_5 b

Log Exposition

FIG_5-d

FIG_5-e

FIG_5-g

FIG_5-f

IMAGE B

FIG_5-h

FIG_5-i

FIG_6

```
        ┌──────────────────┐
        │  IMAGE ORIGINALE │
        │    NUMERISEE     │
        └──────────────────┘
              │ D0 ╮ 21
        ┌─────┼────────────────────────┐ ╮ 24
        │ ┌──────────────────┐         │
        │ │  FILTRAGE MEDIAN │         │
        │ │     SPATIAL      │ ╮ 241   │
        │ └──────────────────┘         │
        │        ╮ 25                  │
        │ ┌──────────────────┐         │
        │ │     FILTRAGE     │         │
        │ │  PAR MOYENNE     │ ╮ 242   │
        │ │    SPATIALE      │         │
        │ └──────────────────┘         │
        └──────────────────────────────┘
                    ╮ 26
        ┌──────────────────┐
        │    VALEURS        │ ╮ 27
        │   ABSOLUES        │
        └──────────────────┘
              ╮ 70
        ┌──────────────────┐
        │  FILTRAGE PAR     │
        │ MOYENNE MOBILE    │ ╮ 71
        └──────────────────┘
        ┌──────────────────────────────┐
        │ ┌──────────────────┐         │
        │ │ HISTOGRAMME DU BRUIT│ ╮ 72 │
        │ └──────────────────┘         │
        │ ┌──────────────────┐         │
        │ │    RECHERCHE     │         │
        │ │  DE CRETE DE     │ ╮ 72    │
        │ │   POPULATION     │         │
        │ └──────────────────┘         │
        │ ┌──────────────────┐         │
        │ │ FILTRAGE MEDIAN  │         │
        │ │  EN DENSITE      │ ╮ 73    │
        │ │    OPTIQUE       │         │
        │ └──────────────────┘         │
        │ ┌──────────────────┐         │
        │ │  FILTRAGE PAR    │         │
        │ │  MOYENNE EN      │ ╮ 74    │
        │ │ DENSITE OPTIQUE  │         │
        │ └──────────────────┘         │
        └──────────────────────────────┘
        ┌──────────────────┐
        │     COURBE        │ ╮ 33
        │ CARACTERISTIQUE   │
        └──────────────────┘
        ┌──────────────────┐
        │ CARTE DES PENTES  │ ╮ 29
        └──────────────────┘
```

```
        ┌──────────────────────────────┐ ╮ 76
        │ ┌──────────────────┐         │
        │ │    FILTRAGE      │         │
        │ │   FERMETURE      │         │
        │ └──────────────────┘ ╮ 68    │
        │ ┌──────────────────┐         │
        │ │  SOUSTRACTION     │        │
        │ └──────────────────┘ ╮ 67    │
        │  TOP HAT                      │
        └──────────────────────────────┘
                    ╮ 28
        ┌──────────────────┐
        │       B / A       │ ╮ 69
        └──────────────────┘
              ╮ 60
```

Bruit

## FIG_7-a

30
31

Dtm    D0

Nb(Dtm)

## FIG_7-b

Atc    Bruit

Bruit

## FIG_7-c

Atc

d

Dtm    D0

Bruit

## FIG_7-d

17

Dtm    D0

FIG_8